(19) Europäisches Patentamt / European Patent Office / Office européen des brevets

(11) **EP 2 055 248 B1**

(12) **EUROPEAN PATENT SPECIFICATION**

(45) Date of publication and mention
of the grant of the patent:
**23.11.2011  Bulletin 2011/47**

(51) Int Cl.:
***A61B 17/16*** *(2006.01)*    ***A61F 2/46*** *(2006.01)*

(21) Application number: **08167458.2**

(22) Date of filing: **23.10.2008**

(54) **Opto-electric indicators for orthopaedic instruments**

Optoelektrische Anzeigen für orthopädische Instrumente

Indicateurs optoélectroniques pour instruments orthopédiques

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.10.2007  US 926414**

(43) Date of publication of application:
**06.05.2009  Bulletin 2009/19**

(73) Proprietor: **DePuy Products, Inc.**
**Warsaw, IN 46581 (US)**

(72) Inventor: **Sherman, Jason T**
**Warsaw, IN 46580 (US)**

(74) Representative: **Belcher, Simon James**
**Urquhart-Dykes & Lord LLP**
**Tower North Central**
**Merrion Way**
**Leeds LS2 8PA (GB)**

(56) References cited:
**WO-A-2006/039483      US-A- 5 893 848**
**US-A1- 2006 173 381      US-A1- 2007 249 901**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to instruments for use in orthopaedic surgery.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joint prostheses usually include components formed from metal, ceramic or plastic materials that are fixed to existing bone.

Such joint replacement surgery is otherwise known as joint arthroplasty. Joint arthroplasty is a well-known surgical procedure by which a diseased and/or damaged joint is replaced with a prosthetic joint. In a typical total joint arthroplasty, the ends or distal portions of the bones adjacent to the joint are resected or a portion of the distal part of the bone is removed and the artificial joint is secured thereto.

There are known to exist many designs and methods for manufacturing implantable articles, such as bone prostheses. Such bone prostheses include components of artificial joints such as elbows, hips, knees and shoulders.

In order to implant the prosthesis, many different instruments may be used. In some instances an instrument, such as a reamer, may include visual markings to aid in the preparation of the bone or insertion of the implant. For example, a reamer may be used to cut holes in the bone or a channel into a long bone. When using a reamer, it is important to only remove the necessary amount of bone. Therefore, many reamers currently in use are often provided with markings to indicate the depth that the bone has been reamed. However, in many existing reamers, the markings are etched onto the metal side and can be difficult for the operator to see while using the reamer.

In other instruments, mechanicals stops are sometimes used to solve this problem. When the reamer gets to the appropriate depth, the stop would effectively prevent the reamer from reaming any further. However, mechanical stops can be difficult and costly to machine. Also, if the stops are adjustable, the stops can sometimes be cumbersome and confusing to use.

WO-2008/039483A2 discusses a bone drill system with highly visible depth drill markings. The depth markers use a pigment which either fluoresces or phosphoresces under UV light to provide contrast with the rest of the drill.

US-2007/0249901 relates to endoscopes and discusses sensing the depth of insertion of a endoscope with an external device that detects sensors or transponders provided on the endoscope shaft.

In one aspect, the invention provides an instrument for use in implanting a joint prosthesis into a bone as defined in appended claim 1. The instrument includes a rod having a proximal end and a distal end. The proximal end includes a visual indicator. The instrument also includes a housing. The rod is moveable in a longitudinal direction relative to the housing. The visual indicator is capable of emitting light when the instrument is in use. As the rod moves in the longitudinal direction, the visual indicator enters into the housing, causing the light emitted to disappear.

The instrument can be used in a method for providing a visual indicator on an instrument for use in implanting a joint prosthesis into a bone is provided. The method includes providing the instrument. The instrument includes a housing and a rod having a visual indicator. The rod is slidably coupled with the housing. The method also includes using the instrument such that the visual indicator emits a light and sliding the rod into the housing until the visual indicator is in the housing. The light emitted by the visual indicator disappears from view.

In one embodiment, the visual indicator emits light when the instrument is in use. As the rod moves in the longitudinal direction, the visual indicator enters into the housing, causing the visual indicator to cease emitting light.

Embodiments of the invention are described below by way of example with reference to the accompanying drawings, in which:

FIG. 1 is a front view of a reamer in position in a long bone and having a visual indicator according to one embodiment of the present invention;

FIG. 2 is a front view of the reamer of FIG. 1 with the visual indicator in the housing;

FIG. 3 is a front view of a reamer in position in a long bone and having a plurality of visual indicators according to one embodiment of the present invention

FIG. 4 is a circuit diagram of the visual indicator according to one embodiment of the present invention; and

FIG. 5 is a front view of a reamer in position in a long bone and having a visual indicator according to one embodiment of the present invention;

FIG 6 is a front view of a reamer in position in a long bone and having a plurality of visual indicators according to one embodiment of the present invention; and

FIG. 7 is a flow chart describing the operation of the reamer of FIG. 1 according to one embodiment of the present invention.

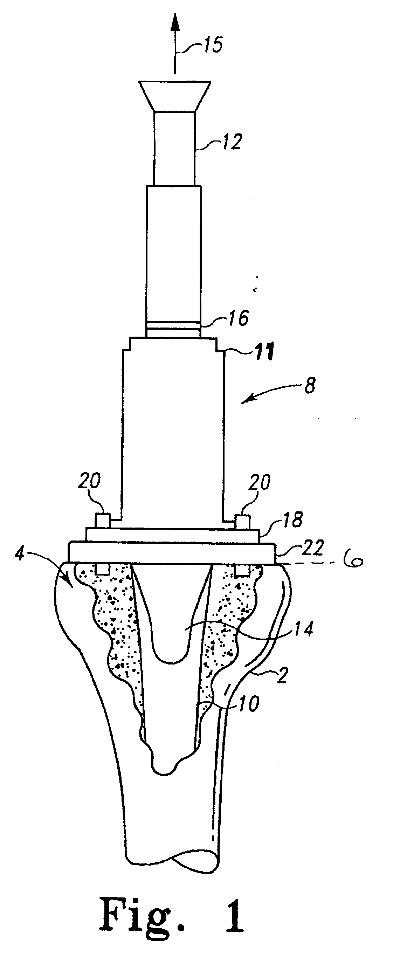

Referring to the drawings, FIG. 1 show a long bone 2, such as a femur or tibia. The long bone 2 includes an intramedullary canal 4 into which a prosthesis may be inserted. The long bone 2 is resected along resection line 6 by, for example, a power tool, for example, a saw. Resection of the long bone 2 exposes the intramedullary canal 4 of the long bone 2. A reamer 8 according to one embodiment of the present invention is positioned in the intramedullary canal 4 of the long bone 2 to form cavity 10 for receiving an orthopaedic joint implant. The reamer 8 is used to remove bone and other biological matter from the intramedullary canal 4 to form the cavity 10. The reamer 8 is rotated by use of a connector (not shown) positioned on the reamer 8. The connector may be any standard connector for example a Hudson or an A-O connector. The connector is used to connect to a power tool (not shown) for rotating the reamer 8. The power tool (not shown) may be any standard power tool. It should be appreciated that the reamer 8 may be rotated through the use of the connector by a hand tool for example a "T" shaped handle.

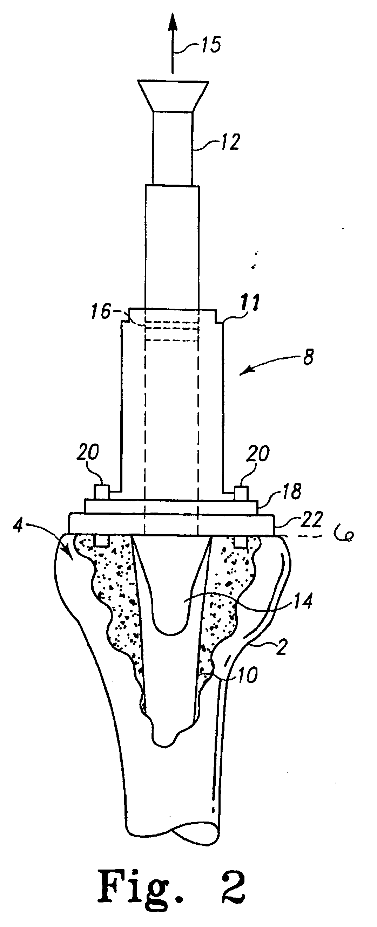

As shown in FIG. 1, the reamer 8 includes a housing 11 and a rod 12 that extends through the housing 11 and includes the cutting edges 14 of the reamer 8. The rod 12 is moveable along a longitudinal direction 15 relative to the long bone 2 and the housing 11. The rod 12 also includes a visual indicator 16. The visual indicator 16 may be an LED or other light source that is capable of shining a beam of light above the housing 11. The light from the visual indicator 16 will provide the user with the ability to know when to stop reaming. Once the light is no longer seen, the user can stop the reamer 8. The operation of the visual indicator will be discussed more below in reference to FIG. 2.

The housing 11 of the reamer 8 includes a base 18. The base 18 includes a plurality of pins 20 that couple the base 18 to a plate 22 and the bone 2. The pins 20, base 18, and plate 22 ensure that the reamer 8 is securely fixed to the bone 2 and that there is not any relative movement between the housing 11 and the bone 2, to ensure a more accurate reaming of the bone.

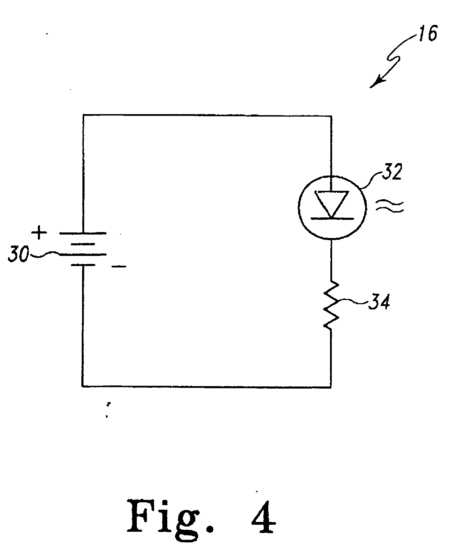

Operation of the visual indicator 16 will be described with reference to FIGS. 1 and 2. Prior to the reamer 8 beginning reaming, the visual indicator 16 will be above the housing 11. In one embodiment, the visual indicator 16 will include a switch that will be activated by the rotation of the rod 8 about the longitudinal axis 15. The switch (not shown) may be a button that is activated by the user. Alternatively, the switch may be a centrifugal switch that is actuated by the rotating of the reamer 8. For example, the centrifugal switch may include a spring that extends outwardly when the reamer 8 begins to spin, completing the circuit (shown without a switch in FIG. 4). Once the reamer 8 stops spinning, the spring would retract, breaking the contact, thereby turning off the light.

As the cutting edges 14 move downward into the long bone 2, the visual indicator 16 also moves downward, eventually moving below the housing 11 as shown in phantom in FIG. 2. Once the visual indicator 16 is below the housing 11, the light it emits is no longer visible. Alternatively, the act of entering the housing may turn the visual indicator 16 off, so that no more light is emitted. Regardless of the method, the user would not see the light any more and would know to stop reaming.

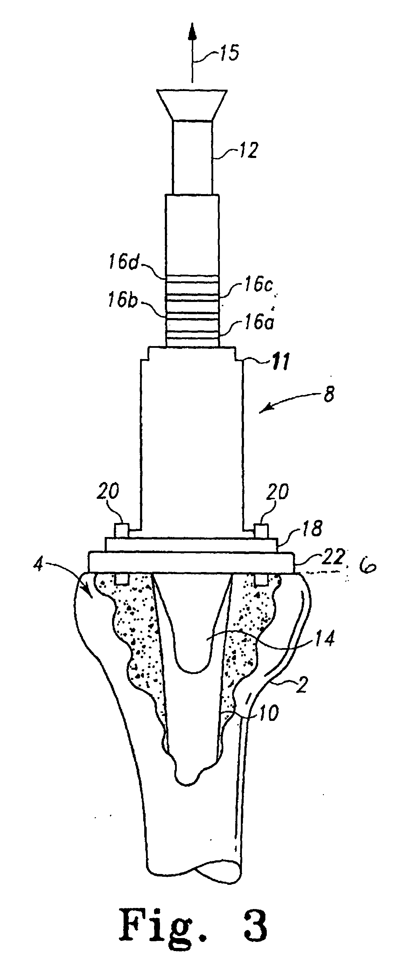

As shown in FIG. 3, some embodiments may include multiple visual indicators 16a, 16b, 16c, 16d. Each visual indicator 16a, 16b, 16c, 16d will emit a different colored light. The spacing of the various visual indicators 16a, 16b, 16c, 16d will indicate different reaming depths. The user (such as the surgeon) will first determine how far the bone 2 needs to be reamed and which color light corresponds to that depth. Once the user begins reaming, all of the visual indicators 16a, 16b, 16c, 16d will emit light. Then, as the rod 12 moves downward, the visual indicators 16a, 16b, 16c, 16d will, one-by-one, descend below the housing 11. Correspondingly, the lights from the visual indicators 16a, 16b, 16c, 16d will, one-by-one, disappear. Once the colored light that corresponds to the depth the user wants to ream can no longer be seen, the user knows to cease reaming.

Turning now to FIG. 4, a circuit diagram of the visual indicator 16 according to one embodiment of the present invention is illustrated. In this embodiment, the visual indicator 16 is a battery-powered LED. As shown, the visual indicator includes a battery 30 connected in series to an LED 32 and to a resistor 34. The positive terminal of the battery 30 is connected to the anode of the LED 32. The cathode of the LED 32 is then connected to the resistor 34, which is in turn connected to the negative terminal of the battery 30. The formula to use to calculate the correct resistor 34 to use is:

$$ R = \frac{V_p - V_l}{I_l} $$

where:

    $V_p$ is the voltage of the power supply (such as a 9 volt battery)
    $V_l$ is the voltage drop across the LED
    $I_l$ is the manufacturer rating of the LED

Generally, the voltage drop depends on the color and the brightness of the LED, for example as set out in the following table.

EP 2 055 248 B1

| Diode type | Typical voltage drop (volts) |
|---|---|
| non-high-brightness red | 1.7 |
| high-brightness, high-efficiency and low-current red | 1.9 |
| orange and yellow | 2.0 |
| green | 2.1 |
| bright white, bright non-yellowish green, and most blue | 3.4 |
| bright blue and some specialized LEDs | 4.6 |

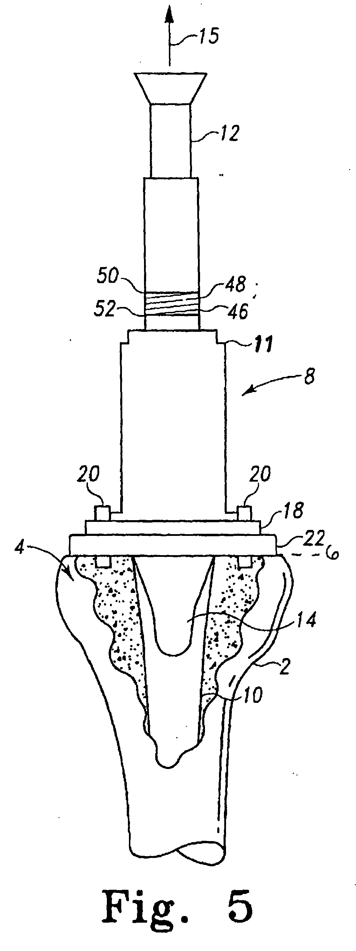

Turning now to FIG. 5, another embodiment of the present invention will be illustrated. As in FIGS. 1 to 3, the reamer 8 includes a housing 11 and a rod 12. The rod 12 includes a visual indicator 46. In this embodiment, the visual indicator 46 is a light collecting mechanism, such as a long strand of scintillating ambient light collecting fiber optic filament 48, wrapped around the circumference of the rod 12. The rod 12 includes two lips or ridges 50, 52 for keeping the filament 48 in place. The lips 50, 52 may also include outwardly extending protrusions to hold the filament 48 against the rod 12.

The filament 48 collects ambient light over a large surface area and emits an intensified beam of light at the end of the filament 48, as known in the art. Similar to the embodiment described in reference to FIGS. 1 and 2, as the rod 12 (and the visual indicator 46) is lowered into the housing 11, the filament 48 will stop emitting light (or the light will no longer be able to be seen by the user). Because the user will not be able to see the light, the user will know to stop reaming.

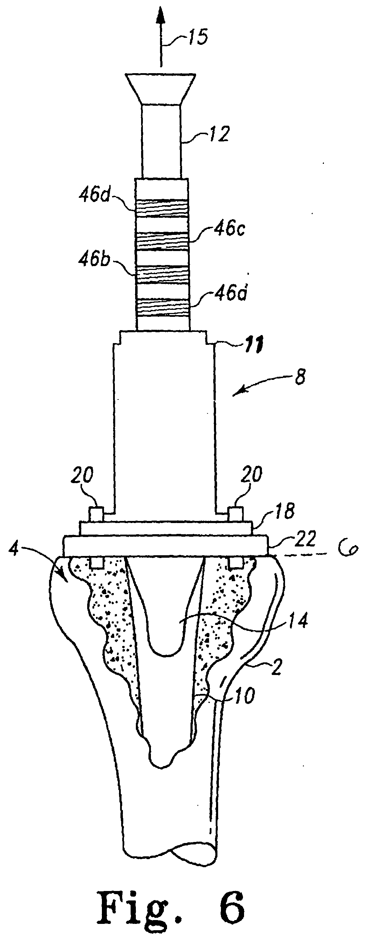

As shown in FIG. 6, the reamer 8 may also include a plurality of light indicators 46a, 46b, 46c, 46d, with each indicator emitting a different color light and corresponding to a different reamer depth.

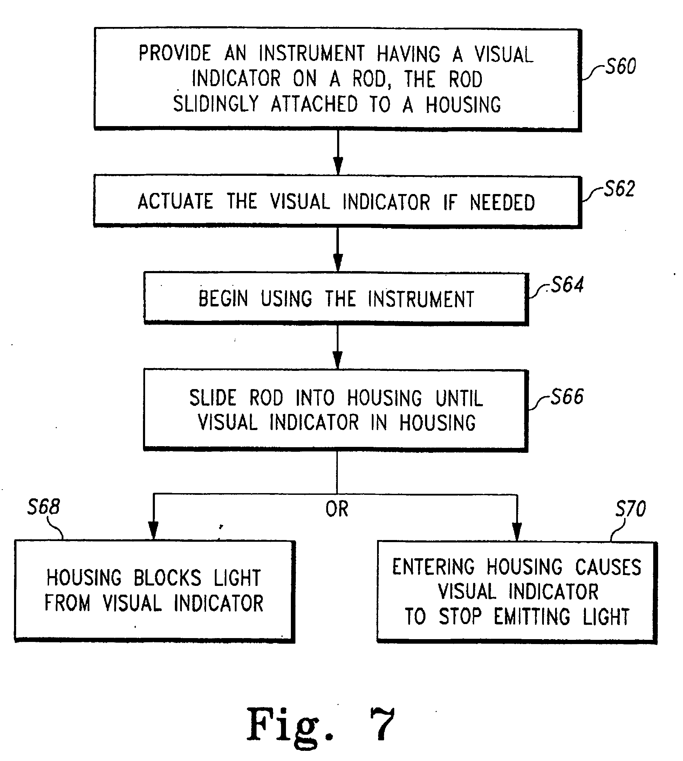

Turning now to FIG. 7, a flow chart illustrating the operation of one embodiment of the present invention is shown. An instrument including a visual indicator on a rod that is slidingly coupled to a housing is provided at step s60. In some embodiments, the user may need to activate the visual indicator at step s62. In other embodiments, such as with the light collecting fiber, the user may not need to activate the visual indicator, and the method may proceed to step s64, in which the user begins to use the instrument. The rod is slid into the housing at step s66 until the visual indicator is within the housing. Then, either the housing blocks the light that is emitting from the visual indicator (step s68) or the act of entering the housing causes the visual indicator to stop emitting light (s70).

Although the invention has been described above in terms of its use on reamers, it can be implemented with other types of instruments.

**Claims**

1. An instrument for use in implanting a joint prosthesis into a bone, the instrument comprising:

a rod (12) having a proximal end and a distal end, the proximal end including a visual indicator (16, 46) capable of emitting light when the instrument is in use; and **characterised in that** the instrument further comprises a housing (11), the rod (12) moveable in a longitudinal direction (15) relative to the housing (11), in which as the rod (12) moves in the longitudinal direction (15), the visual indicator (16, 46) enters into the housing, causing the light emitted to disappear.

2. The instrument of claim 1, in which the rod (12) includes a plurality of visual indicators (16a-d, 46a-d), each of the visual indicators emitting a different color of light.

3. The instrument of claim 2, in which each of the plurality of visual indicators (16a-d, 46a-d) are separated by a predetermined distance, such that as the rod (12) is moved in the longitudinal direction (15), the plurality of visual indicators (16a-d, 46a-d) enter the housing (11) one-by-one.

4. The instrument of claim 1, in which the instrument is a reamer (8) and the distal end of the rod (12) includes a cutting edge for cutting the bone and the visual indicator (16, 46) corresponds to a depth of the bone to be cut.

5. The instrument of claim 4, in which reamer (8) cuts by rotating the rod (12) about a longitudinal axis and the visual indicator (16, 46) is activated by the rotation of the rod (12).

6. The instrument of claim 1, in which the visual indicator (16, 46) is one of an LED (32) and a light collecting fibre optic (48).

7. The instrument of claim 6, in which the visual indicator (16) is an LED (32) and includes a switch that is activated by the use of the instrument and is de-activated once the LED (32) enters the housing (11).

8. The instrument of claim 1, wherein the visual indicator (16, 46) emits light when the instrument is in use, in which as the rod (12) moves in the longitudinal direction (15) the visual indicator (16, 46) enters into the housing (11), causing the visual indicator (16, 46) to cease emitting light.

9. The instrument of claim 8, in which the visual indicator (16) is an LED (32) and includes a switch that is activated when the instrument is in use.

10. The instrument of claim 9, in which the switch is de-activated when the visual indicator (16) enters the housing (11).

11. The instrument of claim 8, in which the visual indicator (46) is a scintillating ambient light collecting fibre optic filament (48) that ceases to emit light when the visual indicator (46) enters the housing (11).

12. The instrument of claim 8, in which the visual indicator (16, 46) is a plurality of visual indicators (16a-d, 46a-d), each of the plurality of visual indicators emitting a different colour of light.

13. The instrument of claim 12, in which each of the plurality of visual indicators (16a-d, 46a-d) corresponds to a measurement of the instrument.

14. The instrument of claim 8, in which the instrument is a reamer (8) and the distal end of the rod (12) includes a cutting edge for cutting the bone and the visual indicator (16, 46) corresponds to a depth of the bone to be cut.

**Patentansprüche**

1. Instrument zur Implantation einer Gelenkprothese in einen Knochen, wobei das Instrument umfasst:

eine Stange (12) mit einem proximalen Ende und einem distalen Ende, wobei das proximale Ende einen optischen Anzeiger (16, 46) enthält, der Licht emittieren kann, wenn das Instrument eingesetzt wird; und **dadurch gekennzeichnet, dass** das Instrument ferner umfasst
ein Gehäuse (11), wobei die Stange (12) in der Längsrichtung (15) relativ zum Gehäuse (11) beweglich ist, wobei, wenn sich die Stange (12) in der Längsrichtung (15) bewegt, der optische Anzeiger (16, 46) in das Gehäuse eintritt, wodurch das emittierte Licht verschwindet.

2. Instrument nach Anspruch 1, wobei die Stange (12) eine Vielzahl von optischen Anzeigern (16a-d, 46a-d) enthält, wobei jeder der optischen Anzeiger eine andere Lichtfarbe emittiert.

3. Instrument nach Anspruch 2, wobei jeder der Vielzahl von optischen Anzeigern (16a-d, 46a-d) in einem vorab festgelegten Abstand angeordnet ist, so dass, wenn die Stange (12) in der Längsrichtung (15) bewegt wird, die Vielzahl von optischen Anzeigern (16a-d, 46a-d) einer nach dem anderen in das Gehäuse (11) eintreten.

4. Instrument nach Anspruch 1, wobei das Instrument eine Reibahle (8) ist und das distale Ende der Stange (12) eine Schneidkante zum Schneiden des Knochens enthält und der optische Anzeiger (16, 46) einer zu schneidenden Tiefe des Knochens entspricht.

5. Instrument nach Anspruch 4, wobei die Reibahle (8) durch Drehen der Stange (12) um eine Längsachse schneidet und der optische Anzeiger (16, 46) durch die Drehung der Stange (12) aktiviert wird.

6. Instrument nach Anspruch 1, wobei der optische Anzeiger (16, 46) eine LED (32) oder eine Licht sammelnde Faseroptik (48) ist.

7. Instrument nach Anspruch 6, wobei der optische Anzeiger (16) eine LED (32) ist und einen Schalter enthält, der durch die Verwendung des Instruments aktiviert wird und die deaktiviert wird, wenn die LED (32) in das Gehäuse (11) eintritt.

8. Instrument nach Anspruch 1, wobei der optische Anzeiger (16, 46) Licht emittiert, wenn das Instrument eingesetzt wird, wobei, wenn sich die Stange (12) in der Längsrichtung (15) bewegt, der optische Anzeiger (16, 46) in das Gehäuse (11) eintritt, wodurch der optische Anzeiger (16, 46) aufhört, Licht zu emittieren.

9. Instrument nach Anspruch 8, wobei der optische Anzeiger (16) eine LED (32) ist und einen Schalter enthält, der aktiviert wird, wenn das Instrument eingesetzt wird.

10. Instrument nach Anspruch 9, wobei der Schalter deaktiviert wird, wenn der optische Anzeiger (16) in das Gehäuse (11) eintritt.

11. Instrument nach Anspruch 8, wobei der optische Anzeiger (46) ein flimmerndes Umgebungslicht sammelndes Faseroptikfilament (48) ist, das aufhört, Licht zu emittieren, wenn der optische Anzeiger (46) in das Gehäuse (11) eintritt.

12. Instrument nach Anspruch 8, wobei der optische Anzeiger (16, 46) eine Vielzahl von optischen Anzeigern (16a-d, 46a-d) ist, wobei jeder der Vielzahl von optischen Anzeigern eine andere Lichtfarbe emittiert.

13. Instrument nach Anspruch 12, wobei jeder der Vielzahl von optischen Anzeigern (16a-d, 46a-d) einer Messung des Instruments entspricht.

14. Instrument nach Anspruch 8, wobei das Instrument eine Reibahle (8) ist und das distale Ende der Stange (12) eine Schneidkante zum Schneiden des Knochens enthält und der optische Anzeiger (16, 46) einer zu schneidenden Tiefe des Knochens entspricht.

**Revendications**

1. Instrument destiné à être utilisé pour implanter une prothèse articulaire dans un os, l'instrument comprenant :

   une tige (12) ayant une extrémité proximale et une extrémité distale, l'extrémité proximale comprenant un indicateur visuel (16, 46) pouvant émettre de la lumière lorsque l'instrument est utilisé ; et
   **caractérisé en ce que** l'instrument comprend en outre :

   un boîtier (11), la tige (12) mobile dans une direction longitudinale (15) par rapport au boîtier (11), dans lequel lorsque la tige (12) bouge dans la direction longitudinale (15), l'indicateur visuel (16, 46) pénètre dans le boîtier, amenant la lumière émise à disparaître.

2. Instrument selon la revendication 1, dans lequel la tige (12) comprend une pluralité d'indicateurs visuels (16a-d, 46a-d), chacun des indicateurs visuels indiquant une couleur de lumière différente.

3. Instrument selon la revendication 2, dans lequel chacun de la pluralité d'indicateurs visuels (16a-d, 46a-d) est séparé par une distance prédéterminée, de sorte que lorsque la tige (12) est déplacée dans la direction longitudinale (15), la pluralité d'indicateurs visuels (16a-d, 46a-d) pénètrent dans le boîtier (11) un à un.

4. Instrument selon la revendication 1, dans lequel l'instrument est un alésoir (8) et l'extrémité distale de la tige (12) comprend un bord de coupe pour couper l'os et l'indicateur visuel (16, 46) correspond à une profondeur de l'os à couper.

5. Instrument selon la revendication 4, dans lequel l'alésoir (8) coupe en faisant tourner la tige (12) autour d'un axe longitudinal et l'indicateur visuel (16, 46) est activé par la rotation de la tige (12).

6. Instrument selon la revendication 1, dans lequel l'indicateur visuel (16, 46) est l'un parmi une diode électroluminescente (32) et une fibre optique de collecte de lumière (48).

7. Instrument selon la revendication 6, dans lequel l'indicateur visuel (16) est une diode électroluminescente (32) et comprend un interrupteur qui est activé par l'utilisation de l'instrument et est désactivé une fois que la diode électroluminescente (32) pénètre dans le boîtier (11).

8. Instrument selon la revendication 1, dans lequel l'indicateur visuel (16, 46) émet de la lumière lorsque l'instrument est utilisé, dans lequel lorsque la tige (12) se déplace dans la direction longitudinale (15), l'indicateur visuel (16, 46) pénètre dans le boîtier (11), amenant l'indicateur visuel (16, 46) à cesser d'émettre de la lumière.

9. Instrument selon la revendication 8, dans lequel l'indicateur visuel (16) est une diode électroluminescente (32) et comprend un interrupteur qui est activé lorsque l'instrument est utilisé.

10. Instrument selon la revendication 9, dans lequel l'interrupteur est désactivé lorsque l'indicateur visuel (16) pénètre dans le boîtier (11).

11. Instrument selon la revendication 8, dans lequel l'indicateur visuel (46) est un filament de fibre optique de collecte de lumière ambiante scintillante (48) qui cesse d'émettre de la lumière lorsque l'indicateur visuel (46) pénètre dans le boîtier (11).

12. Instrument selon la revendication 8, dans lequel l'indicateur visuel (16, 46) est une pluralité d'indicateurs visuels (16a-d, 46a-d), chacun de la pluralité d'indicateurs visuels émettant une couleur de lumière différente.

13. Instrument selon la revendication 12, dans lequel chacun de la pluralité d'indicateurs visuels (16a-d, 46a-d) correspond à une mesure de l'instrument.

14. Instrument selon la revendication 8, dans lequel l'instrument est un alésoir (8) et l'extrémité distale de la tige (12) comprend un bord de coupe pour couper l'os et l'indicateur visuel (16, 46) correspond à une profondeur de l'os à couper.

# Fig. 1

Fig. 2

**Fig. 3**

Fig. 4

**Fig. 5**

Fig. 6

PROVIDE AN INSTRUMENT HAVING A VISUAL INDICATOR ON A ROD, THE ROD SLIDINGLY ATTACHED TO A HOUSING — S60

ACTUATE THE VISUAL INDICATOR IF NEEDED — S62

BEGIN USING THE INSTRUMENT — S64

SLIDE ROD INTO HOUSING UNTIL VISUAL INDICATOR IN HOUSING — S66

OR

S68 — HOUSING BLOCKS LIGHT FROM VISUAL INDICATOR

S70 — ENTERING HOUSING CAUSES VISUAL INDICATOR TO STOP EMITTING LIGHT

Fig. 7

**EP 2 055 248 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008039483 A2
- US 20070249901 A